# EUROPEAN PATENT APPLICATION

(11) **EP 1 375 652 A2**
(43) Date of publication of application: **02.01.2004**
(21) Application number: 03254087.4
(22) Date of filing: 27.06.2003
(51) Int. Cl.: C12N 13/00, C12N 1/16, A61K 35/72, A61K 41/00

(54) **Oral compositions for HIV-Infected Subjects**

(30) Priority: 28.06.2002 US 184749
(71) Applicant: Ultra Biotech Limited, Douglas IM1 1SA, Isle of Man (GB)
(72) Inventor: Cheung, Ling Yuk, Tai Po Industrial Estate New Terrirories (HK)
(74) Representative: Gardner, Rebecca

(57) **Abstract**

The invention relates to oral compositions comprising yeast cells that can produce a healthful benefit in a subject infected with human immunodeficiency virus. The invention also relates to methods for manufacturing the oral compositions by culturing the yeast cells in a series of electromagnetic fields, and methods of use thereof.

## Description

### 1. FIELD OF THE INVENTION

The invention relates to oral compositions comprising yeast cells that can produce a healthful benefit in a subject infected with human immunodeficiency virus. The invention also relates to methods for manufacturing the oral compositions, and methods of use thereof.

### 2. BACKGROUND OF THE INVENTION

### 2.1 AIDS

AIDS -acquired immunodeficiency syndrome - was first reported in the United States in 1981 and has since become a major worldwide epidemic. AIDS is caused by the human immunodeficiency virus (HIV). By killing or damaging cells of the body's immune system, HIV progressively destroys the body's ability to fight infections and certain cancers. As a result of the weakened immune system, many AIDS patients get life-threatening opportunistic infections, such as pneumonia, retinitis, and aspergillosis.

As of the end of 2000, an estimated 36.1 million people worldwide ― 34.7 million adults and 1.4 million children younger than 15 years - were living with HIV/AIDS. More than 70 percent of these people (25.3 million) live in Sub Saharan Africa; another 16 percent (5.8 million) live in South and Southeast Asia. In 2000 alone, HIV/AIDS associated illnesses caused the deaths of approximately 3 million people worldwide, including an estimated 500,000 children younger than 15 years (UNAIDS. Report on the global HIV/AIDS epidemic: December 2000.)

Approximately 40,000 new HIV infections occur each year in the United States, about 70 percent among men and 30 percent among women. As of the end of 2000, an estimated 322,685 people in the United States were living with AIDS. AIDS is now the fifth leading cause of death in the United States among people aged 25 to 44. See, Centers for Disease Control and Prevention (CDC). HIV/AIDS Surveillance Report 2000; 12(no.2): 1 44. At China's first national AIDS conference in 2001, it was reported that AIDS has spread rapidly in China, which sees an annual 30-percent increase in the number of infections. China had an estimated total number of HIV infections of 600,000 in a 1.3 - billion population

The estimated annual number of AIDS related deaths in the United States fell approximately 67 percent from 1995 to 1999, from 50,877 deaths in 1995 to 16,767 deaths in 1999 (Centers for Disease Control and Prevention (CDC). HIV and AIDS - United States, 1981-2001. MMWR 2001;50:430-434.) The U.S. Food and Drug Administration (FDA) has approved a number of drugs for treating HIV infection. The first group of drugs called reverse transcriptase inhibitors (including nucleoside and non-nucleoside analogs), interrupts an early stage of the virus making copies of itself. Included in this class of drugs are AZT (also known as zidovudine or ZDV), ddC (zalcitabine), ddI (dideoxyinosine), d4T (stavudine), and 3TC (lamivudine), delvaridine (Rescriptor), nevirapine (Viramune), and efravirenz (Sustiva). A second class of drugs called protease inhibitors which interrupt virus replication at a later step in its life cycle have also been approved for use in treatment of AIDS. They include Invirase (saquinavir), Norvir (ritonavir), Crixivan (indinavir), Viracept (nelfinavir), and Agenerase (amprenivir). Because HIV can become resistant to any of these drugs, health care providers must use a combination treatment to effectively suppress the virus. Currently available antiretroviral drugs do not cure people of HIV infection or AIDS. In addition, they all have side effects that can be severe. Some of the nucleoside reverse transcriptase inhibitors may cause a depletion of red or white blood cells, especially when taken in the later stages of the disease. Some may also cause an inflammation of the pancreas and painful nerve damage. The most common side effects associated with protease inhibitors include nausea, diarrhea, and other gastrointestinal symptoms.

Although, these drugs are effective in containing the disease in many cases, the high cost of the drugs is a major hurdle in responding to the AIDS epidemic in China, and in the poor countries of sub-Saharan Africa, where the majority of HIV-positive people live.

While chemotherapeutic agents are useful in the treatment of HIV infections and AIDS, there is a continued need to find treatment modalities and approaches to manage the disease that are more effective and economical, and that have less side effects. The present invention provides an alternative approach to AIDS therapy and management of HIV infection by using an oral composition comprising yeasts.

### 2.2 Yeast-Based Compositions

Yeasts and components thereof have been developed to be used as dietary supplement or pharmaceuticals. However, none of the prior methods uses yeast cells which have been cultured in an electromagnetic field to produce a product that has a beneficial effect in HTV-infected subjects. The following are some examples of prior uses of yeast cells and components thereof:
United States Patent No. 6,197,295 discloses a selenium-enriched dried yeast product which can be used as dietary supplement. The yeast strain *Saccharomyces boulardii sequela* PY 31 (ATCC 74366) is cultured in the presence of selenium salts and contains 300 to about 6,000 ppm intracellular selenium. Methods for reducing tumor cell growth by administration of the selenium yeast product in combination with chemotherapeutic agents is also disclosed.
United States Patent No. 6,143,731 discloses a dietary additive containing whole β-glucans derived from yeast, which when administered to animals and humans, provide a source of fiber in the diet, a fecal bulking agent, a source of short chain fatty acids, reduce cholesterol and LDL, and raises HDL levels.
United States Patent No. 5,504,079 discloses a method of stimulating an immune response in a subject utilizing modified yeast glucans which have enhanced immunobiologic activity. The modified glucans are prepared from the cell wall of Saccharmomyces yeasts, and can be administered in a variety of routes including, for example, the oral, intravenous, subcutaneous, topical, and intranasal route.
United States Patent No. 4,348,483 discloses a process for preparing a chromium yeast product which has a high intracellular chromium content. The process comprises allowing the yeast cells to absorb chromium under a controlled acidic pH and, thereafter inducing the yeast cells to grow by adding nutrients. The yeast cells are dried and used as a dietary supplement.

Citation of documents herein is not intended as an admission that any of the documents cited herein is pertinent prior art, or an admission that the cited documents are considered material to the patentability of the claims of the present application. All statements as to the date or representations as to the contents of these documents are based on the information available to the applicant and does not constitute any admission as to the correctness of the dates or contents of these documents.

### 3. SUMMARY OF THE INVENTION

The present invention relates to biological or oral compositions useful for subjects with HIV infection and/or AIDS. In one embodiment, the present invention provides biological compositions comprising live yeast cells which are capable of producing a healthful benefit in subjects with HIV infection and/or AIDS. In other embodiments, the invention provides methods of making the biological compositions, and methods of using the biological compositions.

In particular, the methods of the invention comprise culturing yeast cells in the presence of a series of electromagnetic fields such that the yeast cells becomes metabolically active. The electromagnetic fields used are each defined by one of five frequency ranges and a broad range of field strength. The starting yeast cells are commercially available and/or accessible to the public, such as but not limited to *Saccharomyces carlsbergensis.* The methods for making the biological compositions of the invention further comprise conditioning or acclimatizing the activated yeast cells in plant extracts and the gastric juice of animals, while in the presence of electromagnetic fields. These methods thus also enable the preparation of activated (and conditioned) yeast cells.

The methods of manufacturing comprise expanding the number of activated and conditioned yeast cells in large scale cultures in the presence of electromagnetic fields, performing quality control measures, and packaging. Pharmaceutical compositions of the invention comprise activated and conditioned yeast cells and one or more pharmaceutically acceptable excipients or carriers. Additional ingredients, such as vitamins and/or flavors may be added to the biological compositions to form the oral compositions of the invention. Such additional carriers and ingredients can improve the healthful benefits, pharmacological properties and organoleptic characteristics of the oral compositions During the manufacturing process, the activated, or activated and conditioned yeast cells may be dried or kept at low temperature, and stored for a period of time.

The biological or oral compositions of the invention are ingested by the subject or used as an additive to be incorporated into food to be consumed by the subject. Dietary supplement and nutritional compositions comprising activated and conditioned yeast cells are encompassed by the invention. Preferably, the subject is a human being.

In various embodiments, the biological or oral compositions of the invention are used to produce a healthful benefit in a subject with HIV infection. In particular, the biological composition of the invention can retard the growth of HIV viruses in an animal which received the composition orally. The composition can also be used to prolong the time of survival of a patient with HIV infection and/or AIDS.

### 4. BRIEF DESCRIPTION OF FIGURES

Fig. 1 Activation and conditioning of yeast cells. 1 yeast cell culture; 2 container; 3 electromagnetic field source; 4 electrode.
Fig. 2. Large scale propagation of yeast cells. 5 first container; 6 second container; 7 third container; 8 yeast cell cultures; 9 electromagnetic field source.

### 5. DETAILED DESCRIPTION OF THE INVENTION

The present invention relates to biological compositions that can produce a healthful benefit in a subject with HIV infection and/or AIDS. The present invention provides methods for manufacturing the biological compositions as well as methods for using the biological compositions.

In one embodiment, the invention provides biological compositions that comprise yeasts. Unlike the traditional use of yeasts as a component of the food, the yeast cells of the invention are not a primary source of nutrients for the subject. Nor are yeast cells used as a carrier of substances, such as metal salts. The yeast cells of the invention are live when administered orally or ingested along with food by a subject. Without being bound by any theory or mechanism, the inventor believes that the culture conditions activate and/or amplified the expression of a gene or a set of genes in the yeast cells such that the yeast cells becomes highly effective in stimulating the animal's immune system, including both specific and non-specific immunological reactions, the results of which are manifested as the overall healthful benefits observed in the treated subject. The healthful benefits provided by using the biological compositions are demonstrated in human patients some of which showed improvements in CD4⁺ cell count and gastrointestinal functions.

In another embodiment, the invention provides methods for making the yeast cells in the biological compositions. The starting materials are normal yeast cells which can readily be obtained commercially or from public microorganism deposits. The methods of the invention comprises a set of culture conditions that can be applied reproducibly to activate the yeast cells. The key feature of the culture conditions used in the methods of the invention is a series of alternating electromagnetic fields of defined frequency ranges and field strengths which are applied to the growing yeast cell culture. The method further comprises the step of conditioning or acclimatizing the activated live yeast cells to the acidic environment of the stomach of the subject. The electromagnetic fields used in these methods can be created reproducibly at various scales, thus enabling even the large scale manufacturing of the biological compositions of the invention. By careful control of the culturing conditions, normal yeast cells can be activated routinely and reproducibly to become yeast cells of the invention.

In yet another embodiment, the invention provides methods for manufacturing an oral composition comprising activated and conditioned yeasts of the invention, and additional ingredients, including but not limited to pharmaceutically acceptable carriers or excipients, vitamins, herb extracts, minerals, amino acids, flavoring agents, coloring agents, or preservatives.

In yet another embodiment, the biological compositions can be added to food which will be consumed by the subject. As known to those skilled in the relevant art, many methods may be used to mix the biological or oral compositions of the invention with food while the yeast cells remain viable. In a particular embodiment, the culture broth comprising live yeast cells of the present invention are added directly to food just prior to consumption. Dried powders of the yeasts can also be reconstituted and added directly to food just prior to consumption.

In various embodiments, the oral compositions of the invention can be consumed directly by a subject or be fed directly to a subject. For example, the subject may drink the culture broth or a fraction thereof that comprises live activated and conditioned yeast cells. Oral compositions comprising dried yeast cells can also be given as a solid dosage form to the subject.

Although it is not necessary, the biological or oral compositions of the invention can be used in conjunction or in rotation with other types of treatment modalities, such as but not limited to chemotherapeutic agents and vaccines. Since the biological compositions of the invention are administered orally, the assistance of health professionals in administration of the composition is generally not essential.

Described below in Section 5.1 are the yeast cells of the invention and methods of their preparation. Section 5.2 describes the use of the biological compositions of the invention a subject suffering from HIV infection and/or AIDS.

### 5.1. PREPARATION OF THE YEAST CELL CULTURES

The yeast cells of the biological composition are produced by culturing a plurality of yeast cells in an appropriate culture medium in the presence of an alternating electromagnetic field over a period of time. The method comprises a first step of activating the yeast cells and a second step of conditioning or acclimatizing the activated yeast cells. The activation process comprises culturing yeast cells in the presence of a series of five electromagnetic fields of specific frequencies and field strength. The activation process during activation allows yeast spores to germinate and yeast cells to grow and divide. The conditioning process comprises further culturing of the activated yeast cells in a medium comprising plant extracts and extracts from the stomach of an animal, in the presence of electromagnetic fields. The activated and conditioned yeast cells can be stored as dried cells after drying the cells under appropriate conditions. The dried activated and conditioned yeast cells can be used later in large scale culturing processes for manufacturing the biological compositions of the invention. The various culturing processes of the invention can be performed either as a batch process or a continuous process.

### 5.1.1 Yeasts

In various embodiments, yeasts of the genera *of Saccharomyces, Candida,* Crebrothecium, *Geotrichum, Hansenula, Kloeckera, Lipomyces, Pichia, Rhodosporidium, Rhodotorula, Torulopsis, Trichosporon,* and *Wickerhamia* can be used in the invention. Generally, fungi used for food manufacturing are preferred.

Non-limiting examples of yeast strains include *Saccharomyces sp.,* AS2.311; *Schizosaccharomyces pombe* Linder, AS2.214, AS2.248, AS2.249, AS2.255, AS2.257, AS2.259, AS2.260, AS2.274, AS2.994, AS2.1043, AS2.1149, AS2.1178, IFFI *1056; Saccharomyces sake* Yabe, ACCC2045; *Saccharomyces uvarum Beijer,* IFFI 1023, IFFI 1032, IFFI 1036, IFFI 1044, IFFI 1072, IFFI 1205, IFFI 1207; *Saccharomyces rouxii* Boutroux, AS2.178, AS2.180, AS2.370, AS2.371; *Saccharomyces cerevisiae* Hansen Var. ellipsoideus, ACCC2043, AS2.2, AS2.3, AS2.8, AS2.53, AS2.163, AS2.168, AS2.483, AS2.541, AS2.559, AS2.606, AS2.607, AS2.611, AS2.612; *Saccharomyces carlsbergensis* Hansen, AS2.162, AS2.189, AS2.200, AS2.216, AS2.265, AS2.377, AS2.417, AS2.420, AS2.440, AS2.441, AS2.443, AS2.444, AS2.459, AS2.595, AS2.605, AS2.638, AS2.742, AS2.745, AS2.748, AS2.1042; *Rhodotorula aurantiaca* (Saito)Ladder; AS2.102, AS2.107, AS2.278, AS2,499, AS2,694, AS2.703, AS2.704 and AS2.1146. Saccharomyces cerevisiae Hansen, ACCC2034, ACCC2035, ACCC2036, ACCC2037, ACCC2038, ACCC2039, ACCC2040, ACCC2041, ACCC2042, AS2.1, AS2.4, AS2.11, AS2.14, AS2.16, AS2.56, AS2.69, AS2.70, AS2.93, AS2.98, AS2.101, AS2.109, AS2.110, AS2.112, AS2.139, AS2.173, AS2.182, AS2.196, AS2.242, AS2.336, AS2.346, AS2.369, AS2.374, AS2.375, AS2.379, AS2.380, AS2.382, AS2.393, AS2.395, AS2.396, AS2.397, AS2.398, AS2.399, AS2.400, AS2.406, AS2.408, AS2.409, AS2.413, AS2.414, AS2.415, AS2.416, AS2.422, AS2.423, AS2.430, AS2.431, AS2.432, AS2.451, AS2.452, AS2.453, AS2.458, AS2.460, AS2.463, AS2.467, AS2.486, AS2.501, AS2.502, AS2.503, AS2.504, AS2.516, AS2.535, AS2.536, AS2.558, AS2.560, AS2.561, AS2.562, AS2.576, AS2.593, AS2.594, AS2.614, AS2.620, AS2.628, AS2.631, AS2.666, AS2.982, AS2.1190, AS2.1364, AS2.1396, IFFI 1001, IFFI 1002, IFFI 1005, IFFI 1006, IFFI 1008, IFFI 1009, IFFI 1010, IFFI 1012, IFFI 1021, IFFI 1027, IFFI 1037, IFFI 1042, IFFI 1045, IFFI 1048, IFFI 1049, IFFI 1050, IFFI 1052, IFFI 1059, IFFI 1060, IFFI l06 IFFI 1202, IFFI 1203, IFFI 1209, IFFI 1210, IFFI 1211, IFFI 1212, IFFI 1213, IFFI 1215, IFFI 1220, IFFI 1221, IFFI 1224, IFFI 1247, IFFI 1248, IFFI 1251, IFFI 1270, IFFI 1277, IFFI 1289, IFFI 1290, IFFI 1291, IFFI 1292, IFFI 1293, IFFI 1297, IFFI 1300, IFFI 1301, IFFI 1302, IFFI 1307, IFFI 1308, IFFI 1309, IFFI 1310, IFFI 1311, IFFI 1331, IFFI 1335, IFFI 1336, IFFI 1337, IFFI 1338, IFFI 1339, IFFI 1340, IFFI 1345, IFFI 1348, IFFI 1396, IFFI 1397, IFFI 1399. Preferred yeast strains include but are not limited to *S. cerevisiae* AS2.501, AS2.502, AS2.503, AS2.504, AS2.535, AS2.558, AS2.560, AS2.561, and AS2.562.

Yeast strains useful for the invention can be obtained from private or public laboratory cultures, or publically accessible culture deposits, such as the American Type Culture Collection, 10801 University Boulevard, Manassas, VA 20110-2209 and the China General Microbiological Culture Collection Center (CGMCC), China Committee for Culture Collection of Microorganisms (CCCCM), Institute of Microbiology, Chinese Academy of Sciences, Haidian, P.O. Box 2714, Beijing, 100080, China.

A non-limiting example of using yeast cells of the invention with *Saccharomyces cerevisiae* Hansen strain AS2.558 is provided in Sections 6 and 7 herein below.

Although it is preferred, the preparation of the yeast cells of the invention is not limited to starting with a pure strain of yeast. The yeast cells in the biological compositions may be produced by culturing a mixture of yeast cells of different species or strains. The constituents of a mixture of yeast cells can be determined by standard yeast identification techniques well known in the art.

In various embodiments of the invention, standard techniques for handling, transferring, and storing yeasts are used. Sterile conditions or clean environments are mandatory for carrying out the manufacturing processes of the invention as the biological compositions are produced for human consumption. The manufacturing process as described herein can be adapted to meet regulatory guidelines on product safety and quality control by standard practice known in the art.

### 5.1.2 Electromagnetic Fields

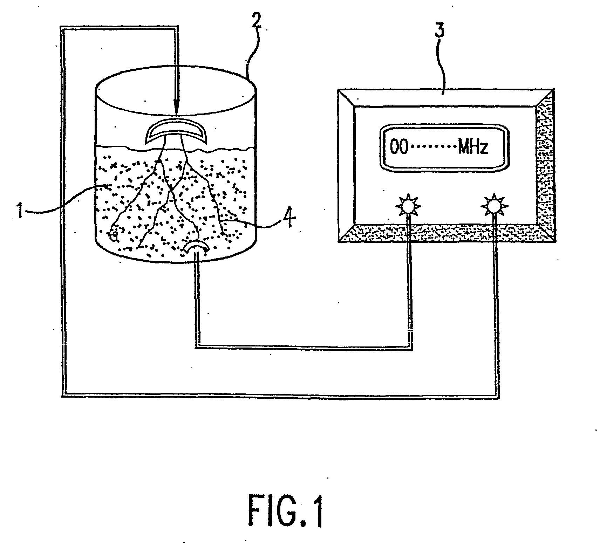

As used herein, the terms "alternating electromagnetic field", "electromagnetic field" or "EM field" are synonymous. An electromagnetic field useful in the invention can be generated by various means well known in the art. A schematic illustration of exemplary setups are depicted respectively in Fig. 1. An electromagnetic field of a desired frequency and a desired field strength is generated by an electromagnetic wave source (3) which comprises one or more signal generators that are capable of generating electromagnetic waves, preferably sinusoidal waves, and preferably in the frequency range of 7000 MHz - 13000 MHz. Such signal generators are well known in the art. Signal generators capable of generating signal with a narrower frequency range can also be used. If desirable, a signal amplifier can also be used to increase the output signal, and thus the strength of the EM field.

The electromagnetic field can be applied to the culture by a variety of means including placing the yeast cells in close proximity to a signal emitter connected to a source of electromagnetic waves. Typically, the yeast cells are placed in a container which is made of material that is not an electric conductor, such as but not limited to plastic, resin, glass, and ceramic.

In one embodiment, the electromagnetic field is applied by signal emitters in the form of electrodes (4) that are submerged in a culture of yeast cells (1). In a preferred embodiment, one of the electrodes is a metal plate which is placed on the bottom of a nonconducting container (2), and the other electrode comprises a plurality of wires or tubes so configured inside the container such that the energy of the electromagnetic field can be evenly distributed in the culture. The electrodes are preferably made of copper. For an upright culture vessel, the tips of the wires or tubes are placed within 3 to 30 cm from the bottom of the vessel (i.e., approximately 2 to 10% of the height of the vessel from the bottom). The number of electrode wires used depends on both the volume of the culture and the diameter of the wire. For example, for a culture having a volume of 10 liter or less, two or three electrode wires having a diameter of between 0.5 to 2.0 mm can be used. For a culture volume of 10 liter to 100 liter of culture, the electrode wires or tubes can have a diameter of 3.0 to 5.0 mm. For a culture volume of 100 liter to 1000 liter, the electrode wires or tubes can have a diameter of 6.0 to 15.0 mm. For a culture having a volume greater than 1000 liter, the electrode wires or tubes can have a diameter of between 20.0 to 25.0 mm.

### 5.1.3 Activation of Yeast Cells

According to the invention, the method for producing activated yeast cells of the invention comprises culturing yeast cells in the presence of a series of five alternating electromagnetic (EM) fields.

The culture process can be initiated by inoculating 100ml of medium with 1ml of an inoculum of a selected yeast strain (such as one of those described in section 5.1.1) at a cell density of about 10⁵ cells/ml. For example, *Saccharomyces cerevisiae* Hansen strain AS2.558 can be used. Preferably, all the culture processes of the invention are carried out under sterile The starting culture can be used to seed larger scale culture. The culture is maintained initially at 28 ° C to 32 ° C for 22 to 30 hours prior to exposure to the EM field(s), typically at 30°C for 27 hours.

The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains sources of nutrients assimilable by the yeast cells. Table 1 provides an exemplary medium for culturing the yeast cells of the invention.

**Table 1**

| Medium Composition | Quantity |
|---|---|
| Glycerol | 20 g |
| Vitamin B2 | 40 µg |
| Vitamin B3 | 40 µg |
| Vitamin B6 | 30 µg |
| Vitamin B12 | 30 µg |
| Vitamin E | 20 µg |
| Vitamin H | 20 µg |
| K₂HPO₄ | 0.20g |
| MgSO₄•7H₂O | 0.22g |
| NaCl | 0.30g |
| CaSO₄•2H₂O | 0.3g |
| CaCO₃•5H₂O | 4.0g |
| Peptone | 2.5g |
| Autoclaved water | 1000ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.2% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are K₂HPO₄, (NH₄)₂HPO₄, CaCO₃, MgSO₄, NaCl and CaSO₄.

It should be noted that the composition of the media provided in Table 1 is not intended to be limiting. The process can be scaled up or down according to needs, and should be carried out under sterile conditions. Various modifications of the culture medium may be made by those skilled in the art, in view of practical and economic considerations, such as the scale of culture and local supply of media components.

In one embodiment, a series of five EM fields are applied to the culture of yeast cells, each having a different set of frequencies within a stated range, and a different set of field strengths within a stated range.

For the first EM field, which can be applied by the apparatus described in section 5.1.2 any means known in the art, the frequency range is 7845 ± 0.5 MHz. The field strength of the EM field is in the range of 270 to 310 mV/cm. The yeast culture is exposed to this first EM field for about 10 to 12 hours, preferably 12 hours.

For the second EM field, the frequency range is 7879 ± 0.5 MHz and the field strength is in the range of 270 to 300 mV/cm. The yeast culture is exposed to this second EM field for about 22 to 28 hours, preferably 25 hours.

For the third EM field, the frequency range is 10141 ± 0.5 MHz and the field strength is in the range of 325 to 366 mV/cm. The yeast culture is exposed to this third EM field for about 22 to 25 hours, preferably 21 hours.

For the fourth EM field, the frequency range is 12842 ± 0.5 MHz and the field strength is in the range of 386 to 412 mV/cm. The yeast culture is exposed to this fourth EM field for about 18 to 26 hours, preferably 22 hours.

For the fifth EM field, the frequency range is 12866 ± 0.5 MHz and the field strength is in the range of 290 to 320 mV/cm. The yeast culture is exposed to this fifth EM field for about 12 to 20 hours, preferably 15 hours.

In less preferred embodiments, the yeast cells can be cultured by exposure to these five EM fields in a different order. In other embodiments, a series of EM fields having field characteristics within the ranges stated above can be applied to activate yeast cells. The yeast cells can remain in the same container and use the same set of electromagnetic wave generator and emitters when switching from one EM field to another EM field. The activated yeast cells may be recovered from the culture by various methods known in the art, and stored at a temperature below about 0°C to 4°C. The activated yeast cells recovered from the liquid culture may be dried and stored in powder form. Preferably, the powder form of the yeast cells comprises greater than about 10¹⁰ yeast cells per gram.

### 5.1.4 Conditioning of Yeast Cells

According to the invention, performance of the activated yeast cells can be optimized by culturing the activated yeast cells in the presence of an extract from the stomach (e.g., the gastric juice) of an animal with physiology similar to the subject to which the biological composition will be administered. The inclusion of this additional conditioning or acclimatizing process allows the activated yeast cells to adapt to and endure the acidic environment of the subject's stomach. The method for conditioning or acclimatizing activated yeast cells of the invention comprises culturing yeast cells in such materials in the presence of a series of two alternating electromagnetic (EM) fields.

The culture process can be initiated by inoculating 1000ml of a conditioning medium with about 10 g of dried activated yeasts containing about 10¹⁰ cells/gram (as prepared by the methods described in section 5.1.3). An equivalent number of yeast cells in culture can also be used as an inoculum. The conditioning medium comprises per 1000 ml about 700 ml of gastric juice of an animal and about 300 ml of wild hawthorn juice. The process can be scaled up or down according to needs. The conditioning process is carried out under sterile conditions.

The gastric juice of an animal can be obtained from the stomach content of a freshly slaughtered animal. The animal is kept under a clean environment, and fed a standard diet, preferably germ-free. For example, the content of the stomach of a 120-day old pig is mixed with 2000 ml of distilled water, and allowed to settle without stirring for 6 hours. The clear liquid above is collected for use as the gastric juice used in the conditioning process. The gastric juice of a pig can be used to condition yeast cells for use in a variety of mammals, including humans. Other methods that can be used to collect the gastric juice include centrifugation or filtration of the mixture to remove debris and/or microorganisms. The gastric juice so obtained can be stored at 4°C. The collection procedures and storage are carried out under sterile conditions.

The wild hawthorn juice is an extract of wild hawthorn fruits prepared by slicing the fruits and drying the splices in air, preferably to less than about 8% moisture (commercial dryer can be used if necessary), crushing the dried fruits to less than about 20 mesh, and mixing 1500 ml of water per 500 gram of the crushed wild hawthorn. The mixture is then allowed to settle without stirring for 6 hours, and the clear liquid above is collected for use as the wild hawthorn juice used in the conditioning process. Other methods that can be used to collect the hawthorn juice include centrifugation or filtration of the mixture. The collection procedures and storage are carried out under sterile conditions.

The activated yeast cells are cultured in a first EM field which can be applied by the apparatus described in section 5.1.2 or any means known in the art. The frequency range of the first EM field is 10141 ± 0.5 MHz. The field strength is in the range of 380 to 420 mV/cm. The temperature is maintained at 28°C to 32°C, and typically at 30°C. The yeast culture is exposed to this first EM field for about 36 to 42 hours, preferably 38 hours.

The activated yeast cells are then cultured in a second EM field which has the following field characteristics: frequency range at 12866 ± 0.5 MHz; field strength at about 340 to 380 mV/cm. The temperature is maintained at 28°C to 32°C, and typically at 30°C. The yeast culture is exposed to this second EM field for about 18 to 28 hours, preferably 22 hours.

In less preferred embodiments, the yeast cells can be cultured by exposing the culture to these EM fields in a different order. In other embodiments, a series of EM fields having field characteristics within the ranges stated above can be applied to condition the yeast cells. The yeast cells can remain in the same container and use the same set of electromagnetic wave generator and emitters when switching from one EM field to another EM field. The activated yeast cells may be recovered from the culture by various methods known in the art, and stored at a temperature below about 0°C to 4°C.

The activated and conditioned yeast cells can be used directly in a biological composition, or used as a starter culture for large scale manufacturing. The activated yeast cells recovered from the liquid culture may be dried and stored in powder form. Preferably, the powder form of the yeast cells comprises greater than about 10¹⁰ yeast cells per gram.

### 5.1.5 Large Scale Manufacturing

The present invention also encompasses methods of manufacturing of the biological compositions of the invention. The activated and conditioned yeast cells as prepared by sections 5.1.3 and 5.1.4 are propagated on a large scale to make the biological compositions of the invention. The method comprises culturing the yeast cells in the presence of one or more EM fields for a period of time, diluting the growing yeast cells with fresh medium, and repeating the process. The method can be carried out as a batch process or a continuous process. The processes of the manufacturing method are carried out under sterile conditions.

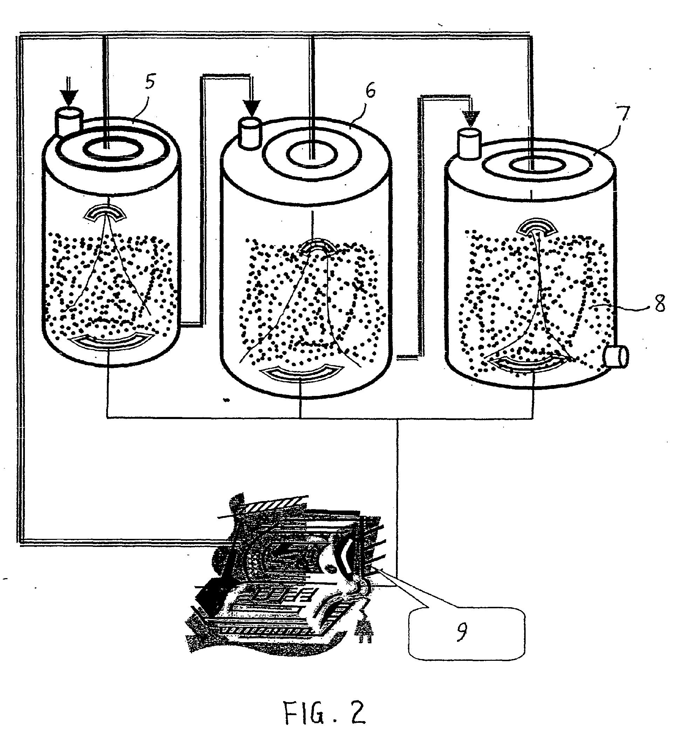

In one preferred embodiment, a set of three containers (5, 6, 7) each comprising a set of electrodes for generating an electromagnetic field as described in section 5.1.2 are set up each with 1000 liters of a culture medium. See Figure 2. The culture medium comprises nutrients assimilable by the yeast cells as shown in Table 2.

**Table 2**

| **Material** | **Quantity** |
|---|---|
| Wild hawthorn juice | 300 liters |
| Jujube juice | 300 liters |
| Wu wei zi juice | 300 liters |
| Soybean juice | 100 liters |

The wild hawthorn juice is an extract of fresh wild hawthorn fruits prepared by washing the fruits clean, drying the fruits in air or using a commerical dryer to less than about 8% moisture, crushing the dried fruits to less than about 20 mesh, and mixing the crushed wild hawthorn with water at a ratio of 2 liters of water per 500 gram of crushed fruits. The mixture is then stirred continuously for 12 hours while the temperature is maintained at 28 °C to 30 °C. The mixture is then centrifuged at 200g x 10 minutes to collect the supernatant which is used as described above. The procedures are carried out under sterile conditions.

The jujube juice is an extract of fresh jujube fruits prepared by washing the fruits clean, drying the fruits to less than about 8% moisture, crushing the dried fruits to less than about 20 mesh, and mixing the crushed jujube with water at a ratio of 2 liters of water per 500 gram of crushed fruits. The mixture is then stirred continuously for 12 hours while the temperature is maintained at 28°C to 30°C. The mixture is then centrifuged at 200g x 10 minutes to collect the supernatant which is used as described above. The procedures are carried out under sterile conditions.

The wu wei zi juice is an extract of fresh berries of *Schisandra chinensis* plant prepared by washing the berries, drying the fruits to less than about 8% moisture, crushing the dried berries to less than about 20 mesh, and mixing the crushed berries with water at a ratio of 2 liters of water per 500 gram of crushed berries. The mixture is then stirred continuously for 12 hours while the temperature is maintained at 28°C to 30°C. The mixture is then centrifuged at 200g x 10 minutes to collected the supernatant which is used as described above. The procedures are carried out under sterile conditions.

The soybean juice is prepared by washing the soybeans, drying the soybeans to less than about 8% moisture, crushing the soybeans to less than about 20 mesh, and mixing the crushed soybeans with water. For 30 kg of soybeans, 130 liters of water is used. The mixture is then stirred continuously for 12 hours while the temperature is maintained at 28°C to 30°C. The mixture is then centrifuged at 200g x 10 minutes to collect the supernatant which is used as described above.

The activated and conditioned yeast cells are cultured in stages, wherein at each stage, the cells are exposed to at least one series of two EM fields:
(h) a field having a frequency in the range of 10141 ± 0.5 MHz and a field strength of 210 to 450 mV/cm; and
(i) a field having a frequency in the range of 12866 ± 0.5 MHz and a field strength of 120 to 400 mV/cm.

The order as stated is preferred.
The culture can be carried out in three containers under sterile conditions.

The first container is inoculated with activated and conditioned yeast cells. About 1000 g of dried yeast powder as prepared by the methods of sections 5.1.3 and 5.1.4 are added to 1000 liter of culture medium. Each gram of the dried yeast powder comprises about 1010 yeast cells. Instead of dried yeast cells, an equivalent number of yeast cells in liquid can also be used.

The yeast cells in the first container (5) are then subjected to a series of two EM fields. For the first EM field, which can be applied by the apparatus described in section 5.1.2, the frequency range is at 10141 ± 0.5 MHz. The field strength of the EM field is in the range of 420 to 450 mV/cm. The yeast culture is exposed to this first EM field for about 16 hours. The yeast cells are then subjected to a second EM field having a frequency of 12866 ± 0.5 MHz and a field strength of about 360 to 400 mV/cm. The yeast culture is exposed to this second EM field for about 12 hours. The yeast cells from the first container are then transferred to the second container which contains about 1000 liter of the culture medium. In effect, the first yeast culture is diluted by about 50% with fresh culture medium.

In the second container (6), the yeast cells are again subjected to a series of two EM fields. The frequencies used in the second container are similar to those used in the first container but the field strengths are marginally lower. The first EM field has the following field characteristics: frequency range at 10141 ± 0.5 MHz; and field strength in the range of 320 to 350 mV/cm. The yeast culture is exposed to this EM field for about 14 hours. The yeast cells are then subjected to a second EM field having a frequency range of 12866 ± 0.5 MHz and a field strength of about 230 to 270 mV/cm. The yeast culture is exposed to this second EM field for about 10 hours. The yeast cells from the second container are then transferred to the third container which contains yet another 1000 liter of the culture medium. Again, the second yeast culture is diluted by about 50% with fresh culture medium.

In the third container (7), the yeast cells are again subjected to a series of two EM fields. The frequencies used in the third container are similar to those used in the first and second container but the field strengths are lower. The first EM field has the following field characteristics: frequency at 10141 ± 0.5 MHz; and field strength in the range of 210 to 250 mV/cm. The yeast culture is exposed to this EM field for about 24 hours. The yeast cells are then subjected to a third EM field having a frequency of 12866 ± 0.5 MHz and a field strength of about 120 to 150 mV/cm. The yeast culture is exposed to this third EM field for about 12 hours.

The yeast cell culture resulting from the end of this stage can be used directly as a biological composition of the invention, or used to form other compositions encompassed by the invention. Other ingredients that enhance the healthful benefits, pharmacological properties and/or organoleptic characteristics of the composition can be added. To maintain viability and freshness of the composition, it is preferred that the various downstream and packaging process be carried out below room temperature, and preferably at 0° to 4°C. Standard methods of quality control and packaging are applied to produce in one embodiment of the invention, oral compositions packaged in liquid containers each comprising about 30 ml or 100 ml of the live yeast cell culture. The concentration of yeast cells is at least 1 x 10³ cells per ml.

In another embodiment, the activated and conditioned yeast cells can be dried as follows. The yeast cell culture is first centrifuged under 75 to 100 g for 10 to 20 minutes to remove the supernantant. The residue which may contain up to 85% moisture is dried in a first dryer at a temperature not exceeding 60 ± 2 ° C for a period of 5 minutes so that yeast cells quickly became dormant. The yeast cells were then sent to a second dryer and dried at a temperature not exceeding 65 ± 2°C for a period of about 8 minutes to further remove water. The dried yeast cells which may contain up to 12% moisture were then cool to room temperature. The dried yeast cells can be packaged by standard pharmaceutical methods in various solid dosage form, each containing a predetermined amount of the dried material. Preferably, the dried material comprises at least IxlO⁶ cells per gram.

In general, the compositions are prepared by uniformly and intimately admixing the active ingredient with liquid carriers or finely divided solid carriers.

### 5.1.6 Preferred Embodiments

In one preferred embodiment, the invention provides a method for preparing a biological composition comprising activated and conditioned yeast cells, said method comprising in any order the steps of:
(a) culturing yeast cells in a first electromagnetic field or series of electromagnetic fields having a frequency of 7845 MHz and a field strength of 295 mV/cm;
(b) culturing the yeast cells in a second electromagnetic field or series of electromagnetic fields having a frequency of 7879 MHz and a field strength of 281 mV/cm;
(c) culturing the yeast cells in a third electromagnetic field or series of electromagnetic fields having a frequency of 10141 MHz and a field strength of 343 mV/cm;
(d) culturing the yeast cells in a fourth electromagnetic field or series of electromagnetic fields having a frequency of 12842 MHz and a field strength of 403 mV/cm; and
(e) culturing the yeast cells in a fifth electromagnetic field or series of electromagnetic fields having a frequency of 12866 MHz and a field strength of 302 mV/cm;
   and after the last of the first five steps, the following steps in any order :
(f) culturing the yeast cells in a liquid medium comprising wild hawthorn juice and gastric juice of a mammal in a sixth electromagnetic field or series of electromagnetic fields having a frequency of 10141 MHz and a field strength of 386 mV/cm; and
(g) culturing the yeast cells in a liquid medium comprising wild hawthorn juice and gastric juice of a mammal in a seventh electromagnetic field or series of electromagnetic fields having a frequency of 12866 MHz and a field strength of 356 mV/cm.

The activated and conditioned yeast cells obtained at the conclusion of this method is encompassed by the invention. Preferably, the yeast cells are *Saccharomyces cerevisiae* strain A52.558. These yeast cells can be used in the following method of further expanding number of activated and conditioned yeast cells.

In another preferred embodiment, the invention provides a method of mass producing a biological composition comprising activated and conditioned yeast cells, said method comprising culturing the activated and conditioned yeast cells prepared by the preferred embodiment described above in this section, in a medium comprising wild hawthorn juice, jujube juice, wu wei zi juice, and soybean juice, and at least one series of electromagnetic fields.

The activated and conditioned yeast cells are cultured in three stages, In a first container (5), the yeast cells are then subjected to a series of two EM fields. For the first EM field, which is applied to the culture for 16 hours, the EM field has a frequency of 10141 MHz and a field strength of 442 mV/cm. For the second EM field which is applied to the culture for 12 hours, the EM field has a frequency of 12866 MHz and a field strength of 384 mV/cm. The order as stated is preferred. The yeast cells from the first container are then transferred to the second container which contains about the same volume of culture medium, thus effectively diluting the culture by about 50%.

In the second container (6), the yeast cells are again subjected to a series of two EM fields. The frequencies used in the second container are similar to those used in the first container but the field strengths are marginally lower. The first EM field has the following field characteristics: frequency at 10141 MHz; and field strength at 335 mV/cm. The yeast culture is exposed to this EM field for about 14 hours. The yeast cells are then subjected to a second EM field having a frequency of 12866 MHz and a field strength of about 252 mV/cm. The yeast culture is exposed to this second EM field for about 10 hours. The yeast cells from the second container are then transferred to the third container which results in a further dilution by about 50% with fresh culture medium.

In the third container (7), the yeast cells are again subjected to a series of two EM fields. The frequencies used in the third container are similar to those used in the first and second container but the field strengths are lower. The first EM field has the following field characteristics: frequency at 10141 MHz; and field strength in the range of 227 mV/cm. The yeast culture is exposed to this EM field for about 24 hours. The yeast cells are then subjected to a second EM field having a frequency of 12866 MHz and a field strength of about 146 mV/cm. The yeast culture is exposed to this second EM field for about 12 hours before harvesting.

### 5.2 Methods of Uses

### 5.2.1 Formulations

The biological compositions of the present invention comprise activated and conditioned live yeast cells prepared as described above, as active ingredient, and can optionally contain a pharmaceutically acceptable carrier or excipient, and/or other ingredients. Other ingredients that can be incorporated into the biological compositions of the present invention, may include, but are not limited to, herbal extracts, vitamins, amino acids, metal salts, metal chelates, coloring agents, flavor enhancers, preservatives, and the like.

In one embodiment, compositions of the present invention suitable for oral administration may be presented as discrete units such as capsules, cachets, or tablets, each containing a predetermined amount of activated and conditioned yeast cells, as a powder or granules, or as a solution or a suspension in an aqueous liquid, a non-aqueous liquid, an oil-in-water emulsion, or a water-in-oil liquid emulsion. In general, the compositions are prepared by uniformly and intimately admixing the active ingredient with liquid carriers or finely divided solid carriers or both, and then, if necessary, shaping the product into the desired presentation. Such products can be used as pharmaceuticals or dietary supplements, depending on the dosage and circumstances of its use.

The oral compositions of the present invention may additionally include binding agents (e.g., pregelatinized maize starch, polyvinylpyrrolidone or hydroxypropyl methylcellulose); binders or fillers (e.g., lactose, pentosan, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets or capsules can be coated by methods well known in the art.

Liquid preparations for oral administration can take the form of, for example, solutions, syrups or suspensions, or they can be presented as a dried product for constitution with water or other suitable vehicle before use. The temperature of the liquid used to reconstitute the dried product should be less than 65 °C. Such liquid preparations can be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). As described below, the preparations can also be made to resemble foods, containing buffer salts, flavoring, coloring and sweetening agents as appropriate

Any dosage form may be employed for providing the subject with an effective dosage of the biological composition. Dosage forms include tablets, capsules, dispersions, suspensions, solutions, capsules, and the like. Generally, because of their ease of administration, tablets and capsules represent the most advantageous oral dosage unit form, in which case solid pharmaceutical carriers as described above are employed. A tablet may be prepared by compression or molding, optionally, with one more accessory ingredients. Tablets may be prepared by compressing in a suitable machine dried activated and conditioned yeast cells in a free-flowing form such as powder or granules, optionally mixed with a binder, lubricant, inert diluent, surface active or dispersing agent. Preferably, the composition is a capsule containing 500 to 700mg of live yeast cells (at greater than about 4 x 10⁵ cells/g) in powder form. In another embodiment, a preferred form of preparation is cell suspension, which contains at least 4 x 10³ yeast cells per ml.

In another embodiment, the biological compositions comprising activated and conditioned yeast cells can be added directly to foods so that an effective amount of yeast cells is ingested during normal meals. Any methods known to those skilled in the art may be used to add to or incorporate the biological compositions into natural or processed foods, provided that the activated and conditioned yeast cells remain viable. Preferably, the nutritional compositions of the invention are made and stored under conditions, such as temperature, from about 0°C to 4°C. As used herein, the term "food" broadly refers to any kind of material, liquid or solid, that is used for nourishing an animal, and for sustaining normal or accelerated growth of an animal including humans. Many types of food products or beverages, such as but not limited to, fruit juice, herbal extracts, tea-based beverages, dairy products, soybean product (e.g., tofu), and rice products, can be used to form nutritional compositions comprising the activated and conditioned yeast cells of the invention.

### 5.2.2 Uses In Subjects with HIV infection and/or AIDS

The present invention further provides methods of use of the biological compositions of the invention. In one embodiment, the biological composition is used as a medicament for treatment of HIV infection and/or AIDS. In another embodiment, the biological composition is used as a dietary supplement, health food, or health drink. The methods comprise administering an effective amount of the biological composition to a subject in need. The biological composition may be administered orally, in liquid or solid form, or enterally through a feeding tube. As used herein, the term "an effective amount" means an amount sufficient to provide a therapeutic or healthful benefit to a subject with HIV infection and/or AIDS.

According to the invention, the biological composition can produce a healthful benefit in a subject suffering from HIV infection and/or AIDS. Preferably, the subject is a human being. The subject in need is one who is diagnosed with HIV infection and/or AIDS, with or without secondary complications, at any stage of the disease. As used herein, the term "AIDS" includes all HIV-infected humans who have fewer than 200 CD4+ T cells per cubic millimeter of blood. (Healthy adults usually have CD4⁺ cell counts of 500-1200 /mm³.) A subject with HIV infection can be diagnosed by testing the subject's blood for the presence of antibodies to HIV by methods known in the art such as ELISA and Western blot analysis. Many of the subjects with advanced HIV disease are inflicted with opportunistic bacterial, viral, fungal, and protozoan infections, which are often severe and sometimes fatal, that generally do not affect healthy people. Non-limiting examples of opportunistic infections include infections by *Candida albicans, Aspergillus fumigatus, Cryptococcus neoformans, Pneumocystis carinii,* and cytomegalovirus. Symptoms of opportunistic infections common in people with AIDS include coughing and shortness of breath, seizures and lack of coordination, difficult or painful swallowing, mental symptoms such as confusion and forgetfulness, severe and persistent diarrhea, fever, vision loss, nausea, abdominal cramps, and vomiting, weight loss and extreme fatigue, severe headaches, and coma. The subjects may also be prone to developing various cancers, especially those caused by viruses such as Kaposi's sarcoma and cervical cancer, or lymphomas.

The subject may have a HIV infection and who is receiving concurrently other treatment modalities against the HIV infection and/or AIDS. The types of secondary infections and recommended treatment vary with the severity of the disease as judged by the CD4⁺ cell count. With a CD4⁺ count above 500/mm³, individualized combination antiretroviral therapy are generally recommended. With a CD4⁺ count at 200 to 500/mm³, there is an increased risk for shingles, thrush, skin infections, bacterial sinus and lung infections, and tuberculosis, and combination antiretroviral therapy are generally recommended for CD4⁺ count < 350//mm³. With a CD4⁺ count 50 to 200/mm³, there is an increased risk for PCP and other opportunistic infections and combination antiretroviral therapy recommended. Prophylaxis for PCP (TMP-SMX, dapsone, or aerosol pentamidine), are necessary and prophylaxis for toxoplasmosis at CD4⁺ count < 100//mm³. When the CD4⁺ count is below 50/mm³, there is an increased risk for opportunistic infections, including MAC and CMV and prophylaxis for MAC (azithromycin, clarithromycin, or rifabutin) becomes necessary.

The subject may be one who has not yet been diagnosed with HIV infection and/or AIDS but are predisposed to or at high risk of contracting HIV and/or developing AIDS as a result of familial factors and/or environmental factors (e.g., blood transfusions, drug abuse, sexual contact with HIV-infected individuals). The subject may also be one who displays characteristics that are associated with HIV infection, such as decline in CD4⁺ T cell count (below 500 /mm³; immunodeficiency) and a flu-like illness accompanied by enlarged lymph nodes.

The subject can be a HIV infected and/or AIDS patient who had undergone a regimen of treatment and whose disease symptoms is regressing or who appears to be clinically free of HIV and/or AIDS symptoms. The biological composition of the invention can be administered adjunctively with any of the treatment modalities, such as but not limited to chemotherapy and immunotherapy. For example, the biological composition can be used while the patient is receiving one or more chemotherapeutic or immunotherapeutic agents, such as nucleoside and non-nucleoside analogs, reverse transcriptase inhibitors, and protease inhibitors. Examples of HIV drugs with which the biological compositions can be used include AZT (also known as zidovudine or ZDV), ddC (zalcitabine), ddI (dideoxyinosine), d4T (stavudine), 3TC (lamivudine), delvaridine (Rescriptor), nevirapine (Viramune), efravirenz (Sustiva), Invirase (saquinavir), Norvir (ritonavir), Crixivan (indinavir), Viracept (nelfinavir), and Agenerase (amprenivir), and combinations thereof. The biological composition can also be used after other regimen(s) of treatment is concluded.

Depending on the subject, the therapeutic and healthful benefits range from inhibiting or retarding the spread of the HIV infection in the body, delaying the onset of opportunistic infections and certain kinds of cancer, palliating the symptoms of the HTV infection and/or AIDS, improving the probability of survival of the subject with HIV infection and/or AIDS, prolonging the life expectancy of the subject, improving the quality of life of the subject, and/or reducing the probability of relapse after a successful course of treatment (operation, and/or chemotherapy). Some of the symptoms associated with HIV infection and/or AIDS are lack of energy, weight loss, frequent fevers and sweats, persistent or frequent yeast infections (oral or vaginal), persistent skin rashes, herpes infections, pelvic inflammatory disease in women, and short term memory loss.

In particular, the invention provides a method for reducing the severity of the HIV infection and/or AIDS in a subject, such as a human, comprising administering orally to the subject a biological composition of the invention. The severity of the disease can be determined by many methods known in the art such as measuring the viral load in circulation, the CD4⁺ T cell count, and by the symptom(s) displayed by the subject. The invention also provide a method for prolonging the time of survival of a subject inflicted with HIV infection and/or AIDS, such as a human patient, comprising administering orally to the subject a biological composition of the invention.

The effective dose for the subject will also vary with the condition to be treated and the severity of the condition to be treated. The dose, and perhaps the dose frequency, will also vary according to the age, body weight, and response of the individual patient. In general, the total daily dose range of activated and conditioned yeast cells is from about 5 x 10⁴ to about 6 x 10⁵ administered in single or divided doses orally. For example, a preferred oral daily dose is two capsules per day (each capsule containing 500 to 700 mg of live yeast celles). For use as a dietary supplement, the oral daily dose should be about 5 x 10⁴ to about 6 x 10⁵. A course of treatment should be at least 13 weeks. It may be necessary to use dosages outside these ranges in some cases as will be apparent to those skilled in the art. The yeast cells can be used for a period of time until the severity of the HIV infection and/or the symptoms of AIDS are reduced or under control, or when the HIV infection and/or AIDS has regressed partially or completely. Further, it is noted that the nutritionist, dietician, clinician or treating physician will know how and when to interrupt, adjust, or terminate use of the biological composition as a medicament or dietary supplement in conjunction with individual patient response.

The effect of the biological compositions of the invention on development and progression of HIV infection and/or AIDS can be monitored by any methods known to one skilled in the art, including but not limited to measuring: virus load, number of CD4⁺ and CD8⁺ cells, etc.

The invention is further defined by reference to the following example describing in detail the clinical trials conducted to study the efficacy and safety of activated and conditioned yeast cells of the invention.

### 6. EXAMPLES

An oral composition of the invention was tested in a small clinical trial involving twenty HIV infected individuals or AIDS patients at the Beijing No. 2 Hospital For Infectious Diseases. The clinicians were Xu Lian Zhi, Wu Hao and Zhang Ke. The subjects were chosen according to the Chinese national AIDS clinical trial standard set up in 1995. Of the 20 subjects, 10 were infected with HIV and the other 10 had AIDS; 12 were male and 8 were female. The ages range from 12 to 45; the average being 28 ± 17. Sixteen subjects contracted HIV by sexual intercourse, 9 were homosexuals; and 4 contracted the virus by blood transfusion. The subjects did not receive other forms of treatment.

The oral composition was prepared using *Saccharomyces cerevisiae* AS2.558 as described in sections 5.1.3 to 5.1.5 above. The activated and conditioned yeast cells were at a concentration of greater than 1 x 10³ per ml. Each subject received 30 ml of the oral composition three times a day, 30 minutes before each meal. The subjects were treated every day for three months. The subjects were checked for the following clinical features: lethargy, appetite, hair loss, diarrhea, weight gain/loss; and safety features: lung and stomach malignancy, vomit, stomach pain, fever, nausea, skin rash, liver and kidney functions, and hematological parameters. The number of CD4⁺ and CD8⁺ cells were also monitored.

The efficacy of the treatment were determined by scores that are based on clinical observations of disease symptoms, CD4⁺ cell count, and other clinical parameters. Using scores based on clinical symptoms, the treatment is deemed efficacious if the ratio of scores after treatment (n2) and scores before treatment (n0) is less than or equal to 0.67; the treatment is deemed to lack efficacy if the ratio of scores after treatment (n2) and scores before treatment (n0) is greater than to 0.67. For scoring purposes, the subjects are divided into two groups based on whether the CD4⁺ cell count was greater than or less than 50 per µl of the subject's blood. For subjects with a CD4⁺ cell count that was greater than 50 per µl, the treatment is deemed efficacious if the cell count increased or continued to remain above 50 per µl; the treatment is deemed to lack efficacy if the cell count fluctuates above and below 50 per µl; the disease worsened if the cell count fell 50 per µl. For subjects with a CD4⁺ cell count that was less than 50 per µl, the treatment is deemed efficacious if the cell count increased or continued to remain above 30 per µl; the treatment is deemed to lack efficacy if the cell count fluctuates above and below 30 per µl; the disease worsened if the cell count fell 30 per µl.

During the three-month trial and at the end of the trial, the subjects did not display any adverse side effects caused by the oral compositions. Before and after the trial, the liver and kidney functions as well as 15 hematological parameters were checked and found to be normal. The results of the trial are shown in Tables 3 and 4.

**Table 3:**

| CD4⁺ cell counts of 8 subjects with a count that was below 50 per µl before treatment, 1 month after treatment and three months after treatment | | | | |
|---|---|---|---|---|
| Patient No. | Before Treatment (per µl) | One month after treatment (per µl ) | Three months after treatment (per µl) | Change in cell count before and after treatment |
| 1 | 2 | 1 | 1 | -1 |
| 7 | 45 | 136 | 93 | 48* |
| 10 | 6 | 30 | 8 | 2 |
| 11 | 17 | 16 | 11 | -6 |
| 12 | 3 | 2 | 0 | -3 |
| 14 | 8 | 11 | 26 | 18* |
| 17 | 7 | 9 | 10 | 3* |
| 20 | 3 | 6 | 12 | 9* |

| | | | | |
|---|---|---|---|---|
| * indicates efficacious treatment | | | | |

**Table 4:**

| CD4⁺ cell counts of 12 subjects with a count that was above 50 per µl before treatment, 1 month after treatment and three months after treatment | | | | |
|---|---|---|---|---|
| Patient No. | Before Treatment (per µl) | One month after treatment (per µl) | Three months after treatment (per µl) | Change in cell count before and after treatment |
| 2 | 73 | 107 | 43 | -30 |
| 3 | 266 | 328 | 405 | 135* |
| 4 | 269 | 434 | 349 | 80* |
| 5 | 250 | 433 | 395 | 145* |
| 6 | 243 | 187 | 180 | -63 |
| 8 | 390 | 389 | 447 | 57* |
| 9 | 378 | 338 | 293 | -85 |
| 13 | 248 | 212 | 303 | 55* |
| 15 | 333 | 226 | 389 | 56* |
| 16 | 140 | 231 | 199 | 59* |
| 18 | 274 | 151 | 326 | 52* |
| 19 | 448 | 460 | 512 | 64* |

| | | | | |
|---|---|---|---|---|
| * indicates efficacious treatment | | | | |

Based on the above results obtained from 12 subjects with greater than 50 CD4⁺ cells per µl, 9 cases were efficacious (75%) and 3 cases lacked efficacy (25%). For subjects with less than 50 CD4⁺ cells per µl, the oral composition was efficacious in 4 cases (50%), and lacked efficacy in the other 4 cases (50%).

**Table 5**

| shows the scores based on clinical observations relating to gastrointestinal functions, such as appetite and diarrhea, made before, during and after treatment. | | | |
|---|---|---|---|
| Patient No. | Before Treatment (n0) | One month after treatment (n1) | 3 months after treatment (n2) |
| 3 | 6 | 6 | 2 |
| 4 | 5 | 5 | 1 |
| 5 | 6 | 6 | 1 |
| 6 | 4 | 4 | 2 |
| 7 | 5 | 2 | 0 |
| 8 | 6 | 2 | 0 |
| 10 | 5 | 4 | 0 |
| 11 1 | 6 | 5 | 3 |
| 12 | 4 | 4 | 1 |
| 13 | 2 | 2 | 1 |
| 14 | 6 | 6 | 0 |
| 17 | 4 | 1 | 0 |
| 20 | 4 | 0 | 0 |
| Total | 63 | 48 | 11 |

The ratio of total scores after treatment and total scores before treatment (n2/n0) is 0.18 which is smaller than 0.67. In the trial, 15 subjects displayed clinical observable symptoms and 13 subjects displayed symptoms related to gastrointestinal functions such as a lack of appetite and diarrhea which improved appreciably after treatment. The treatment was also effective at reducing lethargy. Before treatment, 6 subjects complained of lethargy. After treatment, the subjects reported a noticeable improvement.

In summary, the oral composition of the invention has been shown to be capable of increasing the CD4⁺ cell count and/or improving the gastrointestinal functions of HTV-infected subjects after three months.

The present invention is not to be limited in scope by the specific embodiments described which are intended as single illustrations of individual aspects of the invention, and functionally equivalent methods and components are within the scope of the invention. Indeed various modifications of the invention, in addition to those shown and described herein will become apparent to those skilled in the art from the foregoing description and accompanying drawings. Such modifications are intended to fall within the scope of the appended claims.

All publications, patents and patent applications mentioned in this specification are herein incorporated by reference into the specification to the same extent as if each individual publication, patent or patent application was specifically and individually indicated to be incorporated herein by reference.

## Claims

1. A biological composition comprising activated yeast cells, wherein said yeast cells are prepared by a method comprising in any order the steps of:
(a) culturing yeast cells in a first electromagnetic field or series of electromagnetic fields having a frequency of 7845 ± 0.5 MHz and a field strength of 270 to 310 mV/cm;
(b) culturing the yeast cells in a second electromagnetic field or series of electromagnetic fields having a frequency of 7879 ± 0.5 MHz and a field strength of 270 to 310 mV/cm;
(c) culturing the yeast cells in a third electromagnetic field or series of electromagnetic fields having a frequency of 10141 ± 0.5 MHz and a field strength of 325 to 366 mV/cm;
(d) culturing the yeast cells in a fourth electromagnetic field or series of electromagnetic fields having a frequency of 12842 ± 0.5 MHz and a field strength of 386 to 412 mV/cm; and
(e) culturing the yeast cells in a fifth electromagnetic field or series of electromagnetic fields having a frequency of 12866 ± 0.5 MHz and a field strength of 290 to 320 mV/cm.

2. A biological composition comprising activated and conditioned yeast cells, wherein the yeast cells are prepared by a method comprising in any order the steps of:
(a) culturing yeast cells in a first electromagnetic field or series of electromagnetic fields having a frequency of 7845 ± 0.5 MHz and a field strength of 270 to 310 mV/cm;
(b) culturing the yeast cells in a second electromagnetic field or series of electromagnetic fields having a frequency of 7879 ± 0.5 MHz and a field strength of 270 to 310 mV/cm;
(c) culturing the yeast cells in a third electromagnetic field or series of electromagnetic fields having a frequency of 10141 ± 0.5 MHz and a field strength of 325 to 366 mV/cm;
(d) culturing the yeast cells in a fourth electromagnetic field or series of electromagnetic fields having a frequency of 12842 ± 0.5 MHz and a field strength of 386 to 412 mV/cm; and
(e) culturing the yeast cells in a fifth electromagnetic field or series of electromagnetic fields having a frequency of 12866 ± 0.5 MHz and a field strength of 290 to 320 mV/cm;
and after the last of the first five steps, the following additional steps in any order:
(f) culturing the yeast cells in a liquid medium comprising wild hawthorn juice and gastric juice of a mammal in a sixth electromagnetic field or series of electromagnetic fields having a frequency of 10141 ± 0.5 MHz and a field strength of 380 to 420 mV/cm; and
(g) culturing the yeast cells in a liquid medium comprising wild hawthorn juice and gastric juice of a mammal in a seventh electromagnetic field or series of electromagnetic fields having a frequency of 12866 ± 0.5 MHz and a field strength of 340 to 380 mV/cm.

3. A biological composition comprising activated and conditioned yeast cells, wherein the yeast cells are prepared by a method comprising culturing the activated and conditioned yeast cells of claim 2 in a liquid medium comprising wild hawthorn juice, jujube juice, wu wei zi juice, and soybean juice, and in the presence of at least one series of two electromagnetic fields comprising in any order:
(h) an eighth electromagnetic field having a frequency of 10141 ± 0.5 MHz and a field strength of 210 to 450 mV/cm; and
(i) a ninth electromagnetic field or series of electromagnetic fields having a frequency of 12866 ± 0.5 MHz and a field strength of 120 to 400 mV/cm.

4. The biological composition of claims 1, 2, or 3, wherein the activated, or activated and conditioned yeast cells are at a concentration of about 10³ cells per ml.

5. The biological composition of claims 1, 2 or 3, wherein the activated, or activated and conditioned yeast cells are dried.

6. A composition comprising the activated and conditioned yeast cells of claims 2 or 3, wherein the activated and conditioned yeast cells are packaged in a solid dosage form.

7. The composition of claim 8 comprising about 5 x 10⁵ to 7 x 10⁵ yeast cells per solid dosage form.

8. A pharmaceutical composition comprising the activated and conditioned yeast cells of claims 2 or 3, and a pharmaceutical acceptable carrier.

9. A dietary supplement comprising the activated and conditioned yeast cells of claims 2 or 3, and one or more ingredients selected from the group consisting of vitamins, amino acids, metal chelates, plant extracts, coloring agents, flavor enhancers and preservatives.

10. A nutritional composition comprising the activated and conditioned yeast cells of claims 2 or 3, and a food product selected from the group consisting of a fruit juice-based beverage, a tea-based beverage, a dairy product, a soybean product, and a rice product.

11. A method for preparing a biological composition comprising activated yeast cells, said method comprising in any order the steps of:
(a) culturing yeast cells in a first electromagnetic field or series of electromagnetic fields having a frequency of 7845 ± 0.5 MHz and a field strength of 270 to 310 mV/cm;
(b) culturing the yeast cells in a second electromagnetic field or series of electromagnetic fields having a frequency of 7879 ± 0.5 MHz and a field strength of 270 to 310 mV/cm;
(c) culturing the yeast cells in a third electromagnetic field or series of electromagnetic fields having a frequency of 10141 ± 0.5 MHz and a field strength of 325 to 366 mV/cm;
(d) culturing the yeast cells in a fourth electromagnetic field or series of electromagnetic fields having a frequency of 12842 ± 0.5 MHz and a field strength of 386 to 412 mV/cm; and
(e) culturing the yeast cells in a fifth electromagnetic field or series of electromagnetic fields having a frequency of 12866 ± 0.5 MHz and a field strength of 290 to 320 mV/cm;

12. A method for preparing a biological composition comprising activated and conditioned yeast cells, said method comprising in any order the steps of:
(a) culturing yeast cells in a first electromagnetic field or series of electromagnetic fields having a frequency of 7845 ± 0.5 MHz and a field strength of 270 to 310 mV/cm;
(b) culturing the yeast cells in a second electromagnetic field or series of electromagnetic fields having a frequency of 7879 ± 0.5 MHz and a field strength of 270 to 310 mV/cm;
(c) culturing the yeast cells in a third electromagnetic field or series of electromagnetic fields having a frequency of 10141 ± 0.5 MHz and a field strength of 325 to 366 mV/cm;
(d) culturing the yeast cells in a fourth electromagnetic field or series of electromagnetic fields having a frequency of 12842 ± 0.5 MHz and a field strength of 386 to 412 mV/cm; and
(e) culturing the yeast cells in a fifth electromagnetic field or series of electromagnetic fields having a frequency of 12866 ± 0.5 MHz and a field strength of 290 to 320 mV/cm;
and after the last of the first five steps, the following steps in any order :
(f) culturing the yeast cells in a liquid medium comprising wild hawthorn juice and gastric juice of a mammal in a sixth electromagnetic field or series of electromagnetic fields having a frequency of 10141 ± 0.5 MHz and a field strength of 380 to 420 mV/cm; and
(g) culturing the yeast cells in a liquid medium comprising wild hawthorn juice and gastric juice of a mammal in a seventh electromagnetic field or series of electromagnetic fields having a frequency of 12866 ± 0.5 MHz and a field strength of 340 to 380 mV/cm.

13. A method of making a biological composition comprising activated and conditioned yeast cells, said method comprising culturing the activated and conditioned yeast cells prepared by the method of claim 12 in a medium comprising wild hawthorn juice, jujube juice, wu wei zi juice, and soybean juice, and in the presence of at least one series of two electromagnetic fields comprising in any order:
(h) an eighth electromagnetic field having a frequency of 10141 ± 0.5 MHz and a field strength of 210 to 450 mV/cm; and
(i) a ninth electromagnetic field or series of electromagnetic fields having a frequency of 12866 ± 0.5 MHz and a field strength of 120 to 400 mV/cm.

14. The method of claim 12 or 13, further comprising after the culturing step drying the activated and conditioned yeast cells.

15. The method of claim 14, wherein the drying step comprises (i) drying at a temperature not exceeding 65°C for a period of time such that the yeast cells become dormant; and (ii) drying at a temperature not exceeding 70°C for a period of time to reduce the moisture content to below 5%.

16. Activated yeast cells, wherein said yeast cells are prepared according to the method defined in claim 1.

17. Activated and conditioned yeast cells, wherein said yeast cells are prepared according to the two part method defined in claim 2.

18. Activated and conditioned yeast cells, wherein the yeast cells of claim 17 have further been cultured in a liquid medium comprising wild hawthorn juice, jujube juice, wu wei zi juice, and soybean juice, and in the presence of at least one series of two electromagnetic fields comprising in any order:
(h) an eighth electromagnetic field having a frequency of 10141 ± 0.5 MHz and a field strength of 210 to 450 mV/cm; and
(i) a ninth electromagnetic field or series of electromagnetic fields having a frequency of 12866 ± 0.5 MHz and a field strength of 120 to 400 mV/cm.

19. A method of preparing activated yeast cells comprising steps (a) to (e) of claim 1 in any order.

20. A method of preparing activated and conditioned yeast cells comprising the two part method defined in claim 2.

21. A method of preparing activated and conditioned yeast cells wherein the yeast cells of claim 20 have further been cultured in a liquid medium comprising wild hawthorn juice, jujube juice, wu wei zi juice, and soybean juice, and in the presence of at least one series of two electromagnetic fields comprising in any order:
(h) an eighth electromagnetic field having a frequency of 10141 ± 0.5 MHz and a field strength of 210 to 450 mV/cm; and
(i) a ninth electromagnetic field or series of electromagnetic fields having a frequency of 12866 ±0.5 MHz and a field strength of 120 to 400 mV/cm.

22. A composition obtainable by the method of any one of claims 11 to 15.

23. Yeast cells obtainable by the method of any one of claims 19 to 21.

24. The composition or yeast cells or dietary supplement of any one of claims 1 to 10 or 16 to 23 for use in therapy.

25. The composition, yeast cells, or dietary supplement of any one of claims 2 to 10, 17, 18, 22 or 23 for reducing the severity of HIV infection and/or AIDS in a human.

26. The composition, yeast cells, or dietary supplement of any one of claims 2 to 10, 17, 18, 22 or 23for increasing the CD4⁺ cell count in a human with a HIV infection and/or AIDS.

27. The composition, yeast cells, or dietary supplement of any one of claims 2 to 10, 17, 18, 22 or 23 for prolonging the time of survival of a human with HIV infection.

28. The composition, dietary supplement, method or use of any preceding claim wherein said yeast cells are *Saccharomyces* cells.

29. The composition, dietary supplement, method or use of any preceding claim wherein said *Saccharomyces* cells are *Saccharomyces cerevisiae* cells.

30. The composition, dietary supplement, method or use of any preceding claim wherein said *Saccharomyces cerevisiae* cells are *Saccharomyces cerevisiae* strain AS2.558.
